Europäisches Patentamt

European Patent Office

Office européen des brevets

(19)

(11) Publication number: **0 000 985**
A1

# EUROPEAN PATENT APPLICATION

(12)

(21) Application number: **78300238.9**

(22) Date of filing: **03.08.78**

(51) Int. Cl.²: **A 61 N 1/36, G 08 C 25/00**

(30) Priority: **19.08.77 GB 34918/77**
**19.06.78 US 917138**

(43) Date of publication of application: **07.03.79**
**Bulletin 79/5**

(84) Designated Contracting States: **DE FR GB NL SE**

(71) Applicant: **STIMTECH, INC., 9440 Science Center Drive, Minneapolis Minnesota (US)**

(72) Inventor: **Keller, John Walter, 8600 S.W. 54th Avenue, Miami Florida (US)**

(74) Representative: **Colgan, Stephen James et al, CARPMAELS & RANSFORD 43 Bloomsbury Square, London, W.C.1A, 2RA. (GB)**

(54) **Program alteration security for programmable pacers.**

(57) In order to prevent erroneous interpretations of spurious alterations, a distinct enabling signal is required in order for programming alteration of data signals to be logically processed by the pacer. A magnetic enabling signal is coupled to the patient, during which time pulse width modulated programming alteration bits are separately magnetically coupled to the pacer. The logical combination of both such signals is required for programming changes.

EP 0 000 985 A1

-1-

## "Program Alteration Security for Programmable Pacers"

### Technical Field

This invention relates to implantable body function control apparatus and particularly, but not exclusively, to body tissue stimulating devices such as cardiac pacemakers.

### Background Art

Pacemakers for generating artificial stimulating pulses for the heart, and which may be implanted in the body, are well known. Originally the electrical circuitry for such pacemakers was of analog design, but in recent years digital circuitry has been also employed. A digital approach to pacemakers has led to the evolution of programmable pacemakers - pacemakers having parameters such as pulse rates which are adjustable (programmable) once the pacemaker has been implanted. Programmable pacemakers are described in, for instance, British Specifications 1,385,954 and 1,398,875. Such pacemakers have circuitry to detect and decode signals transmitted outside the body and alter the program accordingly. In British Specification 1,385,954 (claiming priority based on U.S.S.N. 141,694, in turn a parent of U.S.P.N. 3,805,796 to Tenz) the programming is accomplished by means of a magnetic field which is sensed by a magnetic reed switch; the opening and closing of the switch providing programming pulses to a program store. In British Specification 1,398,875 (based on U.S.P.N. 3,833,005 to Wingrove) the programming is by means of radio frequency transmission and reception.

0000985

Many pacemakers are of the demand-type - that is they only supply a stimulating pulse to the heart when a natural heart beat is absent. To accomplish this, demand pacemakers have means for sensing the presence or absence of natural heart beats and for actuating the stimulating pulse as appropriate. One factor of major importance which must not be ignored with demand pacemakers is the influence of noise, and demand pacemakers generally have means for discriminating between a sensed natural beat and sensed signals arising from noise, so as to prevent the pacemaker for considering that the sensed noise represents a natural heart beat and that no stimulating pulse is required.

We believe that noise must similarly be taken into account in programmable body function control apparatus such as pacemakers (whether of the demand-type or otherwise) so that the apparatus does not consider noise as representing data signals for changing the program stored in the apparatus. If, for example, the program can be altered by means of radio frequency transmission and reception, then it is possible that spurious radio frequency signals from an independent source (i.e. noise) could be interpreted by the apparatus as an instruction to change the program.

Disclosure of Invention

In accordance with the present invention, we reduce the possiblity of noise providing unwanted program changes by arranging that the program cannot be changed unless the body function control apparatus receives a coincidence of at least two signals of dissimilar characteristics. The signals of dissimilar characteristics are so chosen that the likelihood of noise having the combined characteristics is remote.

According to the invention there is provided a programmable body function control apparatus having a control means for influencing a function of the body, means for changing at least one characteristic of the control means so that the controlled influence may also be changed, a program store for controlling said changing means, and program detection means for detecting data signals which change the program stored in the program store, said program detection means including means for detecting at least two signals of dissimilar characteristics and for enabling the data signals to change the stored program only when a coincidence of said at least two signals is obtained by the detection means.

Preferably the body function control apparatus is a body tissue stimulator, in which case the control means may comprise a stimulation pulse generator. Typically, the apparatus is an implantable programmable cardiac pacemaker where a change of program can be used to change the characteristics (e.g. rate) of the pacer pulses.

Preferably the program detection means and the program store have digital circuitry, and the means for enabling the data signals to change the stored program includes a coincidence gate such as AND or NOR gate so that the coincident reception of input signals of appropriate logic at the gate is required before programming output signals are provided at the output.

It is normally sufficient to provide for a coincidence of two signals of dissimilar characteristics, one of the signals carrying the data for changing the program and the other signal representing a program "enable" signal.

The signals of dissimilar characteristics can be chosen from a wide selection of possiblities – the aim being to provide a choice which is unlikely to arise as noise. The choice can be made, for example, by selecting signals from very different positions in the electromagnetic spectrum and which can be easily and safely detected by the apparatus when implanted in the body. For example, the data signals can be provided in radio frequency form and the program enable signals in ultrasonic, infrared, or visible light form. With an ultrasonic/RF combination it is desirable to employ relatively high frequency RF to avoid confusion with the ultrasonic frequencies, and with an infrared or visible light/RF combination it is similarly desirable to use RF.

signals of lower frequency. This of course arises from the fact that ultrasound and infrared, visible light are on opposite sides of the radio frequency bands in the electromagnetic spectrum.

A particularly preferred combination of signals of dissimilar characteristics has been obtained by a steady signal provided by a magnet (providing a program enable signal) and pulse-coded signals transmitted with a carrier frequency of about 10KHz (which are employed to alter the stored·program).

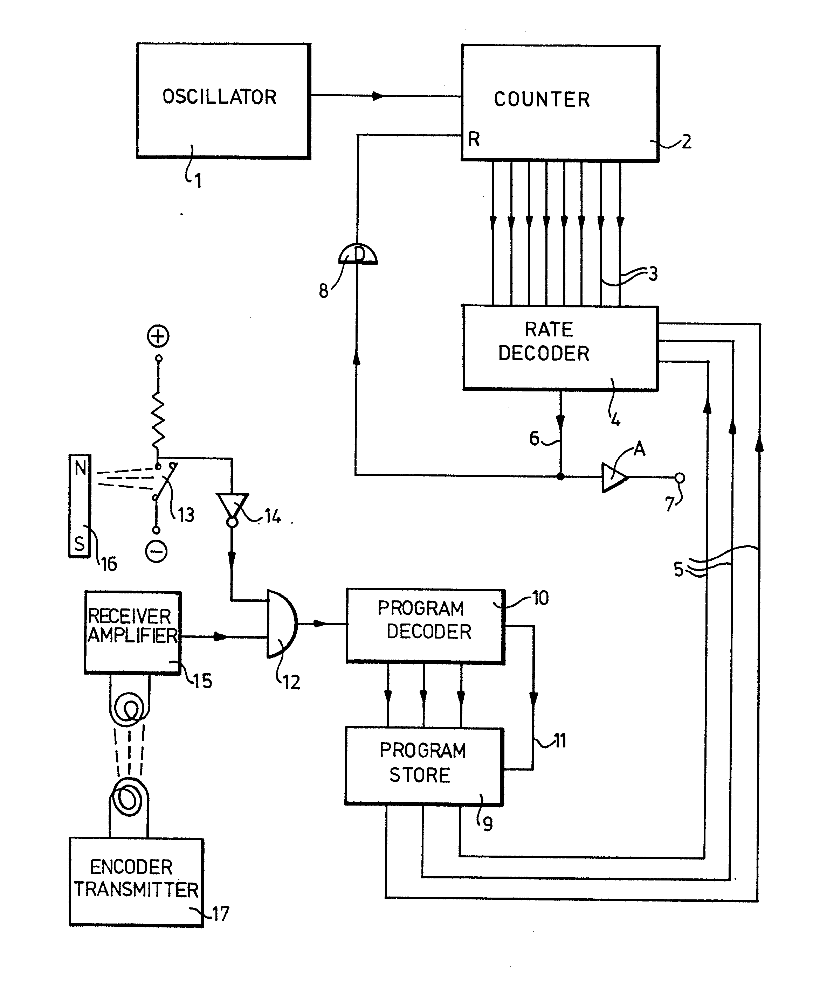

## Brief Description of the Drawing

A preferred embodiment of the invention is illustrated in the accompanying drawing, which shows schematically the electrical circuitry of the body function control apparatus when in the form of an implantable, programmable, cardiac pacemaker.

## Best Mode for Carrying Out the Invention

Referring to the drawing the pacemaker comprises an oscillator 1 which drives a ripple counter 2. The outputs of the various stages of the ripple counter are combined as is known in the art by means of logic gates (not shown) to provide eight output lines 3. The.oscillator frequency and combination of ripple counter outputs are selected so that the eight output lines 3 provide respectively eight different body stimulation pulse frequencies (e.g. 40, 50, 60, 70, 80, 90, 100 and 110 pulses per minute). The eight output lines 3 are supplied to a rate decoder 4 provided with three input control lines 5. By employing binary logic circuitry the logic levels on the lines 5 can be employed to select uniquely one from eight of the eight lines 3 and transmit the selected pulse frequency on line 6. Line 6 is connected via an output amplifier A to a connection 7 leading to a tissue stimulating pulse electrode disposed in or on the heart, and also to a delay unit 8 which, after an appropriate time, resets the counter to enable the next, appropriately timed pulse to be transmitted. The delay provided by delay unit 8 sets the pulse width for each of the stimulating pulses emitted.

The bit values supplied on lines 5 to rate decoder 4 are derived from a three-bit program store 9 whose values are set by corresponding values obtained by a program decoder 10. The binary values held by program decoder 10 are loaded into the program store 9 by means of a strobe program load line 11. The program store 9 and program decoder 10 can be formed in a variety of ways, depending inter alia on the particular form in which data pulses for changing the program are transmitted to the decoder 10 from outside the body.

In the preferred embodiment of the invention the data signals are transmitted by tone burst modulation having a carrier, or modulating frequency of about 10KHz. By "tone burst" modulation we mean pulse width modulation where the pulses of varying width (for bits 0 or 1) modulate a carrier frequency (about 10KHz in our example). A preferred form of program store 9 and decoder 10 for use with tone burst modulated data signals is described in copending Application No. filed on even date (ref: DLD-10) and further details may be obtained therefrom.

The program decoder 10 is fed from an AND gate 12 having two inputs. One input is from a reed switch 13 connected between an electrical source and normally held open, and an inverter 14. The second input to the AND gate 12 is from a program receiver and amplifier 15 which is capable of detecting and amplifying tone burst modulated data pulses transmitted from outside of the body.

The pacemaker operates as follows. With a 3 bit program maintained in the program store 9, the rate decoder 4 selects the appropriate 1 from 8 counts presented by counter 2 in accordance with the logic levels presented on the three input control lines 5. The selected count is presented as a pacing pulse to terminal 7, the counter resets, and the counting pattern is repeated.

If it is desired to change the pacing rate, the program stored by store 9 must be changed to provide a different combination of logic levels on lines 5 to decoder 4. For as long as reed switch 13 remains open, the input to inverter 14 remains high and consequently the corresponding input to AND gate 12 is low thus disabling the latter. In changing the program, a magnet 16 is brought adjacent the patient's body close to the site of the implanted pacemaker. This closes switch 13 and provides a high input on the corresponding line to AND gate 12. Simultaneously, tone burst modulated data signals are transmitted from outside the body from a program encoder and transmitter 17, and are detected and amplified by receiver/amplifier 15. Since the input to AND gate 12 from the reed switch 13 is high, this latter acts as a "program enable" to allow gate 12 to pass the data signals from receiver/amplifier 15 to program decoder 10. A "load the program into the store" signal is then generated on line 11 and the data signals received by decoder 10 clocked into program store 9. The logic levels on lines 5 will then change in accordance with the new stored program to select a different 1 of the 8 counts presented by the counter 2, thus changing the pacemaker pulse rate.

It will be appreciated from this description that it would not be possible to change the stored program unless a coincidence of signals of appropriate logic exists at both inputs to AND gate 12 - this is, the existence of a magnet 16 in sufficiently close proximity to switch 13 to close the latter, coincident with the transmission of tone burst modulated data signals for receipt by receiver/ amplifier 15. Although external noise might be detected by receiver/amplifier 15, the chance that it would occur simultaneously with a magnet being in close proximity to switch 13 is extremely remote and thus the circuit as described acts as a protection against noise being treated as data signals and hence changing the stored program undesirable.

-1-                    0000985

Claims:

1. A programmable body function control apparatus having a control means for influencing a function of the body, means for changing at least one characteristic of the control means so that the controlled influence may also be changed, a program store for controlling said changing means, and program detection means for detecting data signals which change the program stored in the program store, said program detection means including means for detecting at least two signals of dissimilar characteristics and for enabling the data signals to change the stored program only when a coincidence of said at least two signals is obtained by the detection means.

2. An apparatus according to claim 1 wherein said detecting means detects two signals of dissimilar characteristics for enabling.

3. An apparatus according to claim 1 wherein said detecting means includes logic means requiring the logical coincidence of said at least two signals for said enabling.

4. An apparatus according to claim 3 wherein said logic means includes an AND or NOR logic gate.

5. An apparatus according to claim 1 wherein one of said at least two signals represents data for changing said program and another represents a steady program enable signal.

6. An apparatus according to claim 5 wherein said at least two signals are selected from very different positions in the electromagnetic spectrum.

7. An apparatus according to claim 5 wherein one of said at least two signals is a signal in the electromagnetic spectrum for changing said program and another represents a steady program enable signal constituted by a magnetic field.

8. An apparatus according to claim 7 wherein said detecting means includes means for detecting said signal in the electromagnetic spectrum and switch means actuable by said magnetic field.

9. An apparatus according to claim 8 wherein the signal in the electromagnetic spectrum is pulse width modulated.

10. An apparatus according to claim 9 wherein the pulse width modulated signal is additionally tone burst modulated.

11. An apparatus according to claim 1 when in the form of a body tissue stimulator wherein said control means includes a stimulation pulse generator.

12. An apparatus according to claim 11 wherein said body tissue stimulator is an implantable cardiac pacemaker.

13. A cardiac pacemaker according to claim 12 wherein said stimulation pulse generator includes an oscillator and a counter driven by said oscillator, and said changing means includes a rate decoder for selecting a stimulating pulse rate from a plurality of such available from said counter.

## DOCUMENTS CONSIDERED TO BE RELEVANT

| Category | Citation of document with indication, where appropriate, of relevant passages | Relevant to claim |
|---|---|---|
|  | US – A – 3 777 762 (ROVSING)<br>* column 2, lines 12–34; column 4, lines 49–53 * | 1–3,5 –8,11, 12 |
| D | US – A – 3 805 796 (CORDIS)<br>* column 4, lines 31–49; column 5, lines 57–67 * | 3–5, 11,12 |
|  | US – A – 3 945 387 (GEC)<br>* column 8, line 61 to column 9, line 4; column 9, lines 23–35; column 12, lines 23–36 * | 1,3, 11,12 |
| P | US – A – 4 066 086 (MEDTRONIC)<br>* column 3, lines 23–27, 46–61 * | 1–3, 5–8, 11–13 |

**CLASSIFICATION OF THE APPLICATION (Int. Cl.²)**

A 61 N   1/36
G 08 C 25/00

**TECHNICAL FIELDS SEARCHED (Int.Cl.²)**

A 61 N   1/36
A 61 N   1/08
G 08 C 25/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 27–11–1978 | SIMON |